(19) Europäisches Patentamt
European Patent Office
Office européen des brevets

(11) EP 4 710 984 A1

(12) **EUROPEAN PATENT APPLICATION**
published in accordance with Art. 153(4) EPC

(43) Date of publication:
**18.03.2026 Bulletin 2026/12**

(21) Application number: **24825790.9**

(22) Date of filing: **11.06.2024**

(51) International Patent Classification (IPC):
***A61M 25/098*** (2006.01) ***A61L 29/02*** (2006.01)
***A61L 29/06*** (2006.01) ***A61L 29/14*** (2006.01)
***C08K 3/014*** (2018.01) ***C08L 77/00*** (2006.01)

(52) Cooperative Patent Classification (CPC):
**A61L 29/02; A61L 29/06; A61L 29/14; A61M 25/0108; C08K 3/014; C08L 77/00**

(86) International application number:
**PCT/JP2024/021171**

(87) International publication number:
**WO 2024/262385 (26.12.2024 Gazette 2024/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.06.2023 JP 2023102520**

(71) Applicant: **Terumo Kabushiki Kaisha**
**Tokyo 151-0072 (JP)**

(72) Inventor: **WATANABE, Nozomu**
**Fujinomiya-shi, Shizuoka 418-0015 (JP)**

(74) Representative: **KIPA AB**
**Drottninggatan 11**
**252 21 Helsingborg (SE)**

(54) **CATHETER**

(57) There is provided a catheter that is unlikely to deteriorate even after long-term storage. There is provided a catheter including: a catheter tip having a catheter tip resin layer containing 1% by mass or more and less than 50% by mass of tungsten carbide (WC); and a catheter main body portion having a catheter main body portion resin layer containing at least one type of second additive selected from the group consisting of antioxidants and light stabilizers, in which the total content value of at least one type of first additive selected from the group consisting of antioxidants and light stabilizers contained in the catheter tip resin layer is less than the total content value of the second additive in the catheter main body portion resin layer, and the total content value of the first additive relative to the catheter tip resin layer is 0% by mass or more and less than 3% by mass.

*FIG. 2*

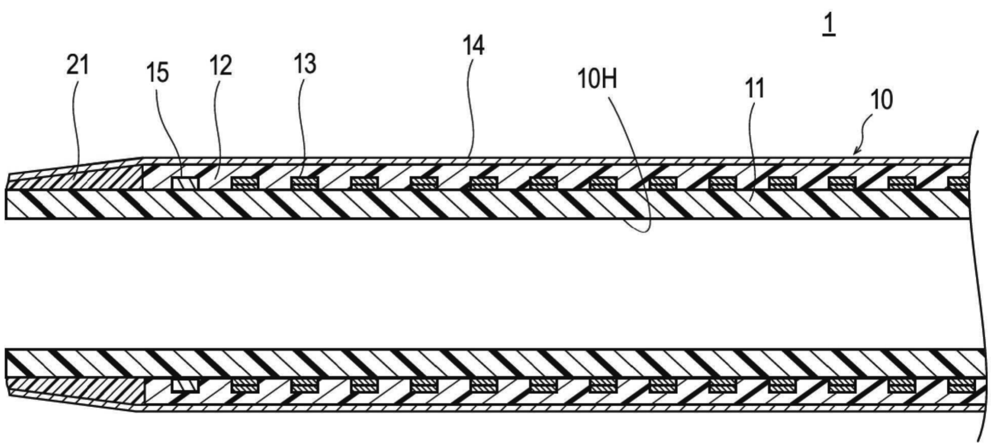


EP 4 710 984 A1

## Description

### Technical Field

The present invention relates to a catheter.

### Background Art

When performing treatment or diagnosis of a stenosed site, a catheter is used to insert a medical device into the living body and guide it to the target location. When inserting a catheter into the living body, in order to accurately identify the position of the distal end of the catheter, the distal end portion of the catheter (catheter tip) typically contains an X-ray opaque material that enables detection by X-rays. For example, metallic tungsten powder is known as an excellent X-ray opaque material and is used as a material for the distal end portion of the catheter (for example, refer to Patent Literature 1).

### Citation List

### Patent Literature

Patent Literature 1: US Patent No. 5584821

### Summary of Invention

Catheters are not always used immediately and may sometimes be stored for a long period before use. Resin is typically used in catheter tips for moldability and flexibility. However, when tungsten is incorporated into the catheter tip as an X-ray opaque material, there is a risk that the resin may deteriorate.
Additionally, in order to prevent damage to blood vessels when inserting the catheter into the living body, the catheter tip, which is the distal end portion, is particularly required to have flexibility.
Accordingly, an object of the present invention is to provide a catheter in which the catheter tip contains an X-ray opaque material, while the tip maintains flexibility and the resin is less likely to deteriorate even after long-term storage.
A catheter according to one embodiment of the present invention has the following configuration.

(1) A catheter including:

a catheter tip having a catheter tip resin layer containing 1% by mass or more and less than 50% by mass of tungsten carbide (WC); and
a catheter main body portion having a catheter main body portion resin layer containing at least one type of second additive selected from the group consisting of antioxidants and light stabilizers,
in which the total content value of at least one type of first additive selected from the group consisting of antioxidants and light stabilizers contained in the catheter tip resin layer is less than the total content value of the second additive in the catheter main body portion resin layer, and
the total content value of the first additive relative to the catheter tip resin layer is 0% by mass or more and less than 3% by mass.

(2) The catheter according to (1), in which the resin contained in the catheter tip resin layer is a resin having ester bonds and/or amide bonds.
(3) The catheter according to (2), in which the resin having ester bonds and/or amide bonds contains a polyamide-based resin.
(4) The catheter according to any one of (1) to (3), in which the particle diameter of the tungsten carbide is 1 to 10 $\mu$m.
(5) The catheter according to any one of (1) to (4), in which the catheter tip does not contain elemental tungsten (W) .
(6) The catheter according to any one of (1) to (5), in which

the antioxidant is at least one selected from phosphorus-based antioxidants, sulfur-based antioxidants, and phenol-based antioxidants, and
the light stabilizer is at least one selected from ultraviolet absorbers and HALS.

(7) The catheter according to any one of (1) to (6), in which the catheter main body portion includes a contrast marker portion.

Brief Description of Drawings

Fig. 1 is a diagram showing an overall configuration of a catheter.
Fig. 2 is a cross-sectional schematic diagram of the vicinity of a junction of a catheter tip and a catheter main body portion.

Description of Embodiments

The present invention provides a catheter including: a catheter tip having a catheter tip resin layer containing 1% by mass or more and less than 50% by mass of tungsten carbide (WC); and a catheter main body portion having a catheter main body portion resin layer containing at least one type of second additive selected from the group consisting of antioxidants and light stabilizers, in which the total content value of at least one type of first additive selected from the group consisting of antioxidants and light stabilizers contained in the catheter tip resin layer is less than the total content value of the second additive in the catheter main body portion resin layer, and the total content value of the first additive relative to the catheter tip resin layer is 0% by mass or more and less than 3% by mass.
According to the catheter of the present invention, it becomes possible to provide a catheter in which the catheter tip has X-ray contrast ability as well as flexibility, and the risk of resin deterioration is reduced even after long-term storage.
Metallic tungsten powder is usually used in combination with a resin. The present inventor speculated that the cause of resin deterioration due to long-term storage may be the hydrolysis of ester bond and/or amide bond sites in the presence of metallic tungsten powder.
Elemental metallic tungsten, due to oxidation by oxygen in the air or during the manufacturing process, is in a state where the oxidation states of metallic tungsten (W) and a very small amount of tungsten oxide ($WO_3$) are mixed, as represented by the following formula. When this $WO_3$ comes into contact with water, it becomes tungstic acid through a hydration reaction and contributes to the release of acidic hydrogen ions (in the present specification, "hydrogen ion" and "acidic hydrogen ion" mean "$H^+$"), that is, acidification.
[Math. 1]

(Formula 1)

$$W + (3/2)O_2 \rightarrow WO_3$$
$$WO_3 + H_2O \rightarrow H_2WO_4 \rightarrow 2H^+ + WO_4^{2-}$$

In fact, a study was conducted to compare the pH values of a resin to which metallic tungsten powder (W) was added (Comparative Example 1, described below), a resin to which tungsten carbide powder (WC) was added (Example 1, described below), and a resin alone, before and after they were subjected to high temperature and high humidity conditions for a certain period. Specifically, 1 g of each of the above three pellet resins was added to 5 mL of RO water and stirred before and after one week of high temperature and high humidity load, and the pH was measured with a pH meter (AS600 manufactured by AS ONE, 25°C) after 24 hours of standing at room temperature. As a result, the pH values of the resin-only sample to which no metal was added and the resin to which tungsten carbide powder of Example 1 was added did not decrease between before and after high temperature and high humidity load, but the pH value of the resin to which metallic tungsten powder of Comparative Example 1 was added decreased significantly before and after high temperature and high humidity load.
Under acidic conditions, ester bond and/or amide bond sites undergo hydrolysis. Furthermore, as hydrolysis proceeds, the terminal carboxyl groups of the degradation products act as acids, which can increase the reaction rate of hydrolysis.
Table 1 below shows the results of molecular weight measurements (PMMA equivalent) using GPC for a sample containing 70% by weight tungsten ((1) pre-loaded sample) and a sample left in a high temperature and high humidity environment for a certain period ((2) loaded sample). Here, in Table 1, Mn means number average molecular weight, Mw means weight average molecular weight, and Mz means z-average molecular weight.

[Table 1]

| Condition | **Mn** | **Mw** | **Mz** | **Mw/Mn** |
|---|---|---|---|---|
| (1) Pre-loaded sample | 9248 | 40418 | 102010 | 4.370 |
| (2) Loaded sample | 4429 | 12164 | 22031 | 2.746 |

From this result, it can be understood that the resin containing metallic tungsten powder undergoes resin decomposition due to high temperature and high humidity load, resulting in a reduction in molecular weight. This decomposition of the resin is considered to be due to hydrolysis of the resin.

The progress of such hydrolysis is considered to gradually proceed even under normal temperature and humidity conditions due to long-term storage of the catheter, potentially leading to aging deterioration of the resin.

Based on this knowledge, the present inventor has studied the matter and found that the use of tungsten carbide (WC) instead of metallic tungsten (W) powder as an X-ray opaque material suppresses resin deterioration under high temperature and high humidity load conditions. The use of tungsten carbide (WC) suppresses the formation of tungsten oxide ($WO_3$) as shown in Formula 1, and the subsequent hydration reaction of tungsten oxide is also less likely to proceed, suppressing the generation of acidic hydrogen ions, thereby suppressing the decrease in pH value, and as a result, it is considered that the hydrolysis of the hydrolyzable resin can be suppressed.

Therefore, the catheter tip may not contain antioxidants/light stabilizers that suppress resin deterioration, and the amount of these additives can be reduced compared to the normally required amount. In addition, the content of tungsten carbide in the catheter tip is within a relatively small range of less than 50% by mass, which secures a certain degree of X-ray contrast ability while maintaining the flexibility of the catheter tip.

Hereinafter, the catheter according to the present invention will be described with reference to the drawings. In the description of the drawings, the same reference numerals will be given to identical elements, and redundant explanations will be omitted. Dimensional ratios in each drawing may be exaggerated for convenience of description, and might be different from actual ratios.

Furthermore, an aspect in which two or three or more of preferable individual aspects of the present invention described below are combined is also regarded as a preferable aspect of the present invention and disclosed in the present specification (that is, it constitutes a legitimate basis for amendment).

Fig. 1 is a diagram showing an overall configuration of a catheter 1 of the present invention. In Fig. 1, the side of the catheter 1 that is inserted into the living body (the left side in Fig. 1) is referred to as the distal end side, the side of the catheter 1 on which a hub 30 is disposed is referred to as the proximal end side, and the direction in which a catheter main body portion 10 of the catheter 1 extends is referred to as the axial direction. Furthermore, in a transverse cross section (cross section orthogonal to the axis) of the catheter main body portion 10 with the axial direction of the catheter main body portion 10 as a reference axis, a direction away from or approaching the catheter main body portion 10 is referred to as a "radial direction".

The catheter 1 includes the catheter main body portion 10 extending in the axial direction, a catheter tip 20 disposed on the distal end side of the catheter main body portion 10, a hub 30 disposed on the proximal end side of the catheter main body portion 10, and a kink-resistant protector (strain relief) 40 disposed between the catheter main body portion 10 and the hub 30. The catheter main body portion 10 and the catheter tip 20 may be joined to each other.

The catheter main body portion 10 is formed of a hollow tubular member provided with flexibility. The catheter main body portion 10 has a lumen 10H over the entire length of the catheter main body portion 10. The lumen 10H is open at a distal end of the catheter tip 20.

Fig. 2 is an enlarged cross-sectional schematic view of a structure of the distal end of the catheter 1 according to one embodiment of the present invention. As shown in Fig. 2, the catheter 1 is configured as a flexible tubular member in which a lumen extending in the axial direction, a distal end opening portion communicating with the lumen, and a proximal end opening portion communicating with the lumen are formed.

The catheter main body portion can form a layered structure to be stacked in the radial direction. The catheter main body portion includes a tubular catheter main body portion resin layer 12 (outer layer), an inner layer 11 disposed on the inner surface of the catheter main body portion resin layer 12, a metal reinforcing body 13 disposed between the catheter main body portion resin layer 12 and the inner layer 11, a contrast marker portion 15 located on the proximal end side of the catheter tip 20 and disposed at the distal end portion of the catheter main body portion, and a hydrophilic lubricating layer 14 disposed on the outer surface of the catheter main body portion resin layer 12. Although not limited in the present invention, in the aspect of Fig. 2, the inner layer 11 and the hydrophilic lubricating layer 14 are disposed extending to the distal end of the catheter tip 20. In other words, in the aspect of Fig. 2, the inner layer 11 and the hydrophilic lubricating layer 14 are common to the inner layer and hydrophilic lubricating layer in the catheter tip 20 and can also form a layer structure with the catheter tip resin layer 21. In addition, in the catheter tip, the catheter main body portion resin layer 12 (without the inner layer 11 and the hydrophilic lubricating layer 14) may consist of a single layer, or the catheter main body portion resin layer 12 may consist of a single layer and contain the metal reinforcing body layer 13 therein.

The inner layer 11 is preferably made of a material such that at least the part in contact with devices such as a treatment catheter or a guide wire, which are inserted into the lumen 10H, has low friction. According to this configuration, a device inserted into the catheter can be moved in the longitudinal direction with lower sliding resistance, thereby improving operability. A specific example of the constituent material for the inner layer 11 includes a fluororesin material such as polytetrafluoroethylene (PTFE).

The catheter main body portion resin layer 12 has a hollow tubular shape extending in the axial direction of the

catheter main body portion 10. The hydrophilic lubricating layer 14 has the same hollow tubular shape as the catheter main body portion resin layer 12, extending in the axial direction of the catheter main body portion 10.

Insertion into the living body becomes smooth when the hydrophilic lubricating layer 14 is provided. The constituent material of the hydrophilic lubricating layer 14 is not particularly limited, but examples thereof include: copolymers of epoxy group-containing monomers such as glycidyl acrylate, glycidyl methacrylate, 3,4-epoxycyclohexylmethyl acrylate, 3,4-epoxycyclohexylmethyl methacrylate, β-methylglycidyl methacrylate, and allyl glycidyl ether, with hydrophilic monomers such as N-methylacrylamide, N,N-dimethylacrylamide, and acrylamide; (co)polymers composed of the above hydrophilic monomers; cellulose-based polymer substances such as hydroxypropyl cellulose and carboxymethyl cellulose; polysaccharides, polyvinyl alcohol, methyl vinyl ether-maleic anhydride copolymers, water-soluble polyamides, poly(2-hydroxyethyl (meth)acrylate), polyethylene glycol, polyacrylamide, polyvinylpyrrolidone, and copolymers of polyvinylpyrrolidone and polyurethane described in U.S. Patent No. 4100309 and Japanese Unexamined Patent Publication No. 59-19582. These hydrophilic lubricating materials may be used singly or in the form of a mixture of two or more types thereof.

The hub 30 is mounted (fixed) to the proximal end of the catheter main body portion 10. In the hub 30, a lumen is formed which communicates with the lumen 10H and in which a Luer taper is formed.

From the hub 30, for example, elongated objects (linear bodies) such as guide wires, catheters (for example, balloon catheters, stent delivery catheters), endoscopes, ultrasound probes, temperature sensors, and the like can be inserted or withdrawn, and various liquids such as contrast agents (X-ray contrast agents), medicinal solutions, and physiological saline can be injected. In addition, the hub 30 can also be connected to other instruments, such as a Y-shaped branch connector. Furthermore, examples of a constituent material for the hub 30 include a thermoplastic resin such as polycarbonate, polyamide, polysulfone, or polyarylate.

Insertion of the catheter into the body is performed while confirming the position thereof under X-ray fluoroscopy. In the present invention, since the catheter tip 20 contains tungsten carbide, which is an X-ray opaque material, the catheter main body portion 10 does not necessarily need to contain X-ray opaque material.

The constituent material for the contrast marker portion 15, which is disposed at the distal end portion of the metal reinforcing body 13 and is provided with X-ray contrast ability, is not particularly limited as long as the material is provided with X-ray contrast ability. For example, metals such as platinum, gold, silver, iridium, titanium, tungsten, or alloys thereof may be suitably used. The contrast marker portion may be a coil or a ring and the like.

In addition, X-ray opaque material (X-ray contrast agent) may also be blended into the constituent material of the catheter main body portion resin layer 12. As the X-ray opaque material, for example, barium sulfate, bismuth oxide, tungsten, or the like can be used. In addition, the X-ray opaque material is not limited to the case of being present over the entire length of the catheter main body portion 10, and may also be present in a part of the catheter main body portion 10, for example, only in the distal end portion of the catheter main body portion 10 or only in the catheter tip 20.

Hereinafter, embodiments for carrying out the present invention will be described in detail. In the present specification, a range of "X to Y" includes X and Y, and indicates "X or more and Y or less". In addition, unless otherwise specified, operations and measurements of physical properties and the like are performed at room temperature (20 to 25°C) and at relative humidity of 40 to 50% RH. A and/or B refers to A, B or a combination of these.

(Catheter)

The catheter 1 of the present invention is an elongated medical device that can be inserted into a biological organ and used for the purpose of treating and improving a stenosed site or can be used for the purpose of delivering a medical instrument such as a stent in a living body. Biological organs include, but are not limited to, blood vessels, bile ducts, trachea, esophagus, other digestive tracts, urethra, nasal cavity and ear cavity, and other organs. In one embodiment, the living organ is a blood vessel, which may be, for example, a coronary artery.

The catheter 1 of the present invention contains an X-ray opaque material. By doing so, when guiding the catheter 1 to a target location through a blood vessel, the catheter 1 can be visualized upon X-ray irradiation by contrast imaging techniques such as X-ray fluoroscopy. The X-ray opaque material may be included particularly in the catheter tip 20.

In one embodiment, the catheter 1 may be a guiding sheath to which a hemostatic valve that is attachable to and detachable from the hub is attached. In one embodiment, the catheter 1 may be a guiding catheter, long sheath, introducer sheath, microcatheter, or guidewire support catheter.

(Catheter Tip)

The catheter tip 20 is formed of a material softer than the catheter main body portion 10. The catheter tip 20 is also referred to as a distal end tip, and has a function of suppressing damage to a lumen in a living body such as a blood vessel and a function of improving insertability into a stenosed site formed in the blood vessel. In Fig. 2, the catheter tip 20 has a tapered shape with a decreasing outer diameter toward the distal end side, but the outer diameter may be identical up to the

distal end side.

The catheter tip has a catheter tip resin layer 21. The catheter tip resin layer 21 includes a resin, preferably a resin having an ester bond and/or an amide bond (resin having an ester bond, an amide bond, or a combination thereof), tungsten carbide, and in some cases, at least one (first additive) selected from the group consisting of an antioxidant and a light stabilizer. Since the catheter tip contains tungsten carbide, the catheter tip has an X-ray contrast ability. The catheter tip resin layer may be a single layer or a plurality of layers of two or more layers. In the case of a plurality of layers, the composition (resin type, mixing ratio, or the like) may be different or the same. Furthermore, the tungsten carbide is preferably dispersed as particles within the resin.

The catheter tip may consist of only the catheter tip resin layer, or other functional layers (for example, the hydrophilic lubricating layer described above on the outer layer) may be laminated to the inner layer side (lumen side) and/or outer layer side (surface side) of the catheter tip resin layer. The hydrophilic lubricating layer is described above.

Resins with ester bond and/or amide bond (hereinafter simply referred to as resins with bonds) include ethylenevinyl acetate copolymers, polyamides, polyamide elastomers, polyurethane elastomers, polyamideimides, polyesters, polyester elastomers and the like. Resins with bonds may be polyamide, polyamide elastomer, polyamideimide, polyester, polyester elastomer and combinations thereof. The above resin may be used singly, or two or more types may be laminated or blended.

Among these, the resin having a bond is preferably one that includes a polyamide-based resin due to its high flexibility and high affinity or low hardness difference with adjacent members; more preferably includes polyamide and/or polyamide elastomer (polyamide, polyamide elastomer, or a combination thereof); and even more preferably includes a polyamide elastomer. Among the resins having a bond, the polyamide-based resin is preferably 50% by mass or more (upper limit: 100% by mass), more preferably 80% by mass or more (upper limit: 100% by mass), and even more preferably 100% by mass (composed of a polyamide-based resin). Further, among the resins having a bond, the polyamide elastomer is preferably 50% by mass or more (upper limit: 100% by mass), more preferably 80% by mass or more (upper limit: 100% by mass), and even more preferably 100% by mass (composed of a polyamide elastomer).

In addition, among the resins constituting the catheter tip, the resin having a bond is preferably 80% by mass or more (upper limit: 100% by mass), more preferably 90% by mass or more (upper limit: 100% by mass), and even more preferably 100% by mass (composed of resin having a bond). Further, among the resin constituting the catheter tip resin layer, the resin having a bond is preferably 80% by mass or more (upper limit: 100% by mass), more preferably 90% by mass or more (upper limit: 100% by mass), and even more preferably 100% by mass (composed of resin having a bond) .

The polyamide elastomer usable as the resin having a bond is a thermoplastic resin composed of a copolymer having a hard segment derived from a crystalline polymer with a high melting point and a soft segment derived from an amorphous polymer with a low glass transition temperature, and refers to one in which the main chain of the polymer forming the hard segment has an amide bond (-CONH-). A structural unit having an amide bond (-CONH-) in the main chain of the polymer forming the hard segment is also referred to as an amide unit of the polyamide elastomer.

The "amide unit of the polyamide elastomer" in the present specification refers to a repeating unit derived from an amide bond in the polyamide elastomer polymer chain, and the amide unit in the polyamide elastomer is preferably a repeating unit represented by the following Formula (1).

[Chem. 1]

Formula (1)

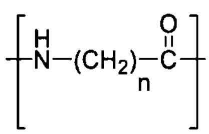

In Formula (1) above, n is preferably an integer of 2 to 20, and more preferably an integer of 5 to 11. Note that n is 11 in the examples described below.

Examples of the polymer forming the soft segment include polyester and polyether. Moreover, examples thereof include polyethers such as polyethylene glycol, polypropylene glycol, polytetramethylene ether glycol (PTMG), and polyester polyol, and ABA-type triblock polyether diols. The polymer forming the soft segment may be used singly or in combination of two or more types thereof. Furthermore, a polyether diamine or the like obtained by reacting ammonia or the like with the terminal of polyether can be used, and for example, an ABA-type triblock polyether diamine can be used. The polyether, which is a polymer capable of forming a soft segment, can form a polyether block amide copolymer in which the polyether block is bonded to the polyamide block, which is a hard segment, via an ester bond.

The content ratio of the soft segment in the polyamide elastomer is preferably 1 to 50% by mass, and more

preferably 10 to 30% by mass.

As the polyamide elastomer, a chain extender such as a dicarboxylic acid may be used in addition to the hard segment and the soft segment.

These polyamide elastomers may be used singly or in combination of two or more types thereof.

The weight average molecular weight of the polyamide elastomer is preferably 10,000 to 500,000, more preferably 15,000 to 400,000, and still more preferably 20,000 to 300,000. In one embodiment, the weight average molecular weight of the polyamide elastomer is preferably 10,000 or more, more preferably 15,000 or more, and still more preferably 20,000 or more. In one embodiment, the weight average molecular weight of the polyamide elastomer is preferably 500,000 or less, more preferably 300,000 or less, and still more preferably 200,000 or less.

In addition, the molecular weight (for example, the number average molecular weight, the weight average molecular weight, and the z-average molecular weight) of the polymer according to the present invention can be measured by a known method such as mass spectrometry, light scattering, liquid chromatography, gas chromatography, or gel filtration chromatography (GPC), and in the present specification, the molecular weight is measured by GPC.

As measurement conditions for the GPC method, for example, the following conditions can be mentioned.

(1) Pretreatment: filtration through a 0.45 μm PTFE cartridge filter
(2) Equipment: HLC-8420GPC (manufactured by Tosoh)
(3) Separation column: TSKgel Super AWM-H (6.0 mm I.D. x 15 cm)
(4) Measurement temperature: 40°C
(5) Carrier: hexafluoroisopropanol (+10 mM $CF_3COONa$)
(6) Flow rate: 0.3 mL/min
(7) Injection volume: 20 μL
(8) Detector: differential refractometer (RI detector), polarity = (+)
(9) Concentration: 1 mg/mL
(10) Molecular weight standard: standard polymethyl methacrylate

The Shore D hardness of the polyamide elastomer used for the catheter tip is preferably 20 to 80 and more preferably 30 to 50 from the viewpoint of flexibility. In the present specification, the method for measuring the hardness of a resin, for example, a polyamide elastomer, uses Shore D hardness in accordance with ISO 868:2003. In the present specification, when a plurality of types of polyamide elastomers are used, Shore D hardness of the whole polyamide elastomer in consideration of the content mass ratio is used.

The polyamide usable as the resin having a bond is not particularly limited as long as the polyamide is a polymer having an amide bond (-CO-NH-) in the main chain, and is typically manufactured by polymerization of a lactam having a cyclic structure or an amino acid, or by polycondensation of a dicarboxylic acid and a diamine. As the polyamide, a homopolyamide is preferably used. Examples of monomers capable of polymerizing alone include ε-caprolactam, ω-laurolactam, 6-aminocaproic acid, enantholactam, 7-aminoheptanoic acid, 11-aminoundecanoic acid, 12-aminododecanoic acid, 9-aminononanoic acid, and piperidone.

In addition, in the polycondensation of a dicarboxylic acid and a diamine, examples of the dicarboxylic acid include adipic acid, sebacic acid, dodecane dicarboxylic acid, glutaric acid, terephthalic acid, 2-methylterephthalic acid, isophthalic acid, and naphthalenedicarboxylic acid. Examples of diamines include tetramethylenediamine, hexamethylenediamine, nonamethylenediamine, decamethylenediamine, undecamethylenediamine, dodecamethylenediamine, para-phenylenediamine, and meta-phenylenediamine.

Examples of the polyamide include NYLON 4, 6, 7, 8, 11, 12, 6.6, 6.9, 6.10, 6.11, 6.12, 6T, 6/6.6, 6/12, 6/6T, and 6T/6I. The polyamide can be used singly or in combination of two or more types thereof.

In addition, the terminal of the polyamide may be capped with a carboxyl group, amine, or the like. Examples of carboxylic acids include aliphatic monocarboxylic acids such as caproic acid, caprylic acid, capric acid, lauric acid, myristic acid, palmitic acid, stearic acid, and behenic acid. Furthermore, examples of the amine include aliphatic primary amines such as hexylamine, octylamine, decylamine, laurylamine, myristylamine, palmitylamine, stearylamine, and behenylamine.

The weight average molecular weight of the polyamide is preferably 10,000 to 500,000, more preferably 15,000 to 400,000, and still more preferably 20,000 to 300,000.

The resin content in the catheter tip resin layer of catheter tip 20 is set appropriately in consideration of X-ray contrast ability, flexibility, and moldability, for example, the resin content may be more than 50% by mass, 55% by mass or more, or 60% by mass or more, and the content of resin in the catheter tip resin layer of the catheter tip 20 is 99% by mass or less, and may be 90% by mass or less, or 85% by mass or less. The resin content in the catheter tip resin layer of catheter tip 20 may be greater than 50% by mass and 99% by mass or less, 55 to 90% by mass, or 60 to 85% by mass. In the present specification, when the catheter tip resin layer consists of a plurality of layers, the content in the catheter tip resin layer of a certain component means the ratio of the total mass value of a certain component in all catheter tip resin layers to the total

mass value of all catheter tip resin layers.

The catheter tip resin layer also contains tungsten carbide as an X-ray opaque material. Metallic tungsten powder, which is commonly used as an X-ray opaque material, can release acid upon oxidation followed by hydration as described above. However, tungsten carbide is superior because it is hardly affected by this phenomenon, and as a result, the risk of resin decomposition is reduced.

In one embodiment, only tungsten carbide is used as the X-ray opaque material. In one embodiment, the catheter tip resin layer contains elemental metallic tungsten (W) powder as the X-ray opaque material. In one embodiment, the catheter tip resin layer does not contain elemental metallic tungsten (W). In one embodiment, the catheter tip contains elemental metallic tungsten(W) powder as the X-ray opaque material. In one embodiment, the catheter tip does not contain elemental metallic tungsten (W). The term "does not contain" in the present specification means, for example, that the material is not intentionally added as a raw material, and is also "not contained" when the material becomes contained due to unintentional contamination, for example, due to decomposition over time. Specifically, the content of metallic tungsten (W) powder is preferably 0.01% by mass or less (lower limit: 0% by mass) and is more preferably 0.001% by mass or less, in the catheter tip resin.

The particle diameter of tungsten carbide can be 1 to 10 μm. When the particle diameter of tungsten carbide is 1 μm or larger, the area is sufficiently small and the area exposed to air is reduced, making it difficult for the decomposition, oxidation, and hydration reactions of the tungsten carbide to proceed, resulting in sufficient suppression of acid release. In addition, when the particle diameter of tungsten carbide is 10 μm or less, the system can be easily homogenized when mixed with resin, contributing to improved operability during manufacturing and maintaining high quality of the catheter tip resin layer. The particle diameter of tungsten carbide is preferably 2 to 5 μm, and more preferably 3 to 4.5 μm. In one embodiment, the particle diameter of the tungsten carbide is 1 μm or more, preferably 2 μm or more, and more preferably 3 μm or more. In one embodiment, the particle diameter of the tungsten carbide is 10 μm or less, preferably 5 μm or less, and more preferably 4.5 μm or less.

Herein, in the present specification, the particle diameter or the particle size refers to the volume average particle diameter, which can be measured, for example, by dynamic light scattering, but is not limited thereto.

In addition, various types of tungsten carbide are commercially available, and these commercial products can be preferably used in the present invention.

Tungsten carbide can be contained in the entire catheter tip resin layer by 1% by mass or more and less than 50% by mass. When the content of tungsten carbide is less than 50% by mass of the total catheter tip resin, flexibility of the catheter tip is secured and a certain degree of X-ray contrast ability is secured. When the content of tungsten carbide is 1% by mass or more relative to the entire catheter tip resin, the contrast ability of the catheter tip under X-rays is improved. Tungsten carbide may be 5% by mass or more, or 10% by mass or more of the entire catheter tip resin layer. Tungsten carbide may be 45% by mass or less, or may be 40% by mass or less of the entire catheter tip resin layer. In addition, tungsten carbide may be 5 to 45% by mass or 10 to 40% by mass of the entire catheter tip resin layer.

In the present invention, the catheter tip resin layer 21 can contain additives. The additives that can be included in the catheter tip resin layer 21 include, without limitation, antioxidants and light stabilizers (in the present specification, at least one type of additive selected from the group consisting of antioxidants and light stabilizers that can be included in the catheter tip resin layer is referred to as the "first additive").

In the present specification, the antioxidant can prevent deterioration such as denaturation or decomposition due to oxidation of the resin. Examples of antioxidants in the present specification include phosphorus-based antioxidants, sulfur-based antioxidants, and phenol-based antioxidants. Specific examples of phosphorus-based antioxidants include, but are not limited to: tris(nonylphenyl) phosphite, tris(2,4-di-t-butylphenyl) phosphite, distearyl pentaerythritol diphosphite, bis(2,4-di-t-butylphenyl) pentaerythritol diphosphite, bis(2,4-dit-butyl-6-methylphenyl) pentaerythritol diphosphite, bis(2,6-di-t-butyl-4-methylphenyl) pentaerythritol diphosphite, bis(2,4-dicumylphenyl) pentaerythritol diphosphite, tetrakis(2,4-di-t-butylphenyl)-4,4'-diphenylenediphosphonite, 2,2'-methylene-bis(4,6-di-t-butylphenyl)-2-ethylhexyl phosphite, 2,2'-ethylidene-bis(4,6-di-t-butylphenyl)fluorophosphite, bis(2,4-di-t-butyl-6-methylphenyl)ethyl phosphite, 2-(2,4,6-tri-t-butylphenyl)-5-ethyl-5-butyl-1,3,2-oxaphosphorinan, 2,2',2"-nitrilotriethyltris(3,3',5,5'-tetra-t-butyl-1,1'-biphenyl-2,2'-diyl)phosphite, and 2,4,8,10-tetra-t-butyl-6-[3-(3-methyl-4-hydroxy-5-t-butylphenyl)propoxy]dibenz[d,f][1,3,2]dioxaphosphepine. Specific examples of sulfur-based antioxidants include, but are not limited to: dilauryl-3,3'-thiopropionate, dimyristyl-3,3'-thiodipropionate, distearyl-3,3'-thiodipropionate, pentaerythritol tetrakis(3-laurylthiopropionate), ditridecyl-3,3'-thiodipropionate, and 1,3,5-tris-β-stearylthiopropionyl oxyethyl isocyanurate. Specific examples of the above phenol-based antioxidants include, but are not limited to: 2,6-di-t-butyl-4-methylphenol, octadecyl-3-(3,5-di-t-butyl-4-hydroxyphenyl)propionate, pentaerythritol tetrakis[3-(3,5-di-t-butyl-4-hydroxyphenyl)propionate], 4,4'-butylidenebis(3-methyl-6-t-butylphenol), ethylenebis(oxyethylene)bis[3-(5-t-butyl-4-hydroxy-m-tolyl)propionate], 4,4'-thiobis(3-methyl-6-t-butylphenol), 1,3,5-trimethyl-2,4,6-tris(3,5-di-t-butyl-4-hydroxybenzyl)benzene, 1,3,5-tris(4-t-butyl-3-hydroxy-2,6-dimethylbenzyl)isocyanurate, 3,9-bis-{2-[3-(3-t-butyl-4-hydroxy-5-methylphenyl)propionyloxy]-1,1-dimethylethyl}-2,4,8,10-tetraoxaspiro[5.5]undecane, tris-(3,5-di-t-butyl-4-hydroxybenzyl)isocyanurate, 2,2'-methylenebis(4-methyl-6-cyclohexylphenol), and 2,2'-dihydroxy-3,3'-di(α-methylcyclohexyl)-5,5'-dimethyldiphenylmethane. These antioxidants may be used singly or in combination of two or more types thereof.

In the present specification, a light stabilizer can prevent deterioration such as denaturation or decomposition caused by radical formation or other reactions resulting from the absorption of light by the resin, by absorbing light of corresponding wavelengths. Examples of light stabilizers in the present specification include, but are not limited to, ultraviolet absorbers and HALS (hindered amine light stabilizers).

Examples of ultraviolet absorbers include, but are not limited to: benzotriazole-based ultraviolet absorbers such as 2-(2-hydroxy-5-methylphenyl)benzotriazole, 2-[2-hydroxy-3,5-bis(α,α-dimethylbenzyl)phenyl]benzotriazole, 2-(2-hydroxy-3-t-butyl-5-methylphenyl)-5-chlorobenzotriazole, 2-(2-hydroxy-3,5-di-t-butylphenyl)-5-chlorobenzotriazole, 2-(2-hydroxy-3,5-di-t-butylphenyl)benzotriazole, 2-(2-hydroxy-3,5-di-t-amylphenyl)benzotriazole, 2-(2-hydroxy-5-t-octylphenyl)benzotriazole, 2-(2-hydroxy-octoxyphenyl)benzotriazole, 2,2'-methylenebis[6-(2H-benzotriazol-2-yl)-4-(1,1,3,3-tetramethylbutyl) phenol], 2-[4,6-bis(2,4-dimethylphenyl)-1,3,5-triazin-2-yl]-5-(octyloxy)phenol, and 2-(4,6-diphenyl-1,3,5-triazin-2-yl)-5-(hexyloxy)phenol; benzophenone-based ultraviolet absorbers such as 2-hydroxy-4-octoxybenzophenone, 2-hydroxy-4-methoxybenzophenone, 2,4-dihydroxybenzophenone, and 2,2',4,4'-tetrahydroxybenzophenone; and benzoate-based ultraviolet absorbers such as 2,4-di-t-butylphenyl 3,5-dit-butyl-4-hydroxybenzoate, and n-hexadecyl 3,5-di-t-butyl-4-hydroxybenzoate.

Examples of HALS include, but are not limited to: bis(2,2,6,6-tetramethyl-4-piperidyl) sebacate, bis(1,2,2,6,6-pentamethyl-4-piperidyl) sebacate and methyl 1,2,2,6,6-pentamethyl-4-piperidyl sebacate (mixture), bis(1,2,2,6,6-pentamethyl-4-piperidyl) [[3,5-bis(1,1-dimethylethyl)-4-hydroxyphenyl]methyl]butyl malonate, decanoic acid bis(2,2,6,6-tetramethyl-1-(octyloxy)-4-piperidyl) ester, reaction product of 1,1-dimethylethyl hydroperoxide and octane, 4-benzoyloxy-2,2,6,6-tetramethylpiperidine, mixture of esters of 2,2,6,6-tetramethyl-4-piperidinol and higher fatty acids, tetrakis(2,2,6,6-tetramethyl-4-piperidyl)-1,2,3,4-butanetetracarboxylate, tetrakis(1,2,2,6,6-pentamethyl-4-piperidyl)-1,2,3,4-butanetetracarboxylate, polycondensate of dimethyl succinate and 4-hydroxy-2,2,6,6-tetramethyl-1-piperidineethanol, poly[{(6-(1,1,3,3-tetramethylbutyl)amino-1,3,5-triazin-2,4-diyl){(2,2,6,6-tetramethyl-4-piperidyl)imino}hexamethylene {(2,2,6,6-tetramethyl-4-piperidyl)imino}}], polycondensate of dibutylamine, 1,3,5-triazine, N,N'-bis(2,2,6,6-tetramethyl-4-piperidyl-1,6-hexamethylenediamine, and N-(2,2,6,6-tetramethyl-4-piperidyl)butylamine, N,N',N",N'''-tetrakis-(4,6-bis-(butyl-(N-methyl-2,2,6,6-tetramethylpiperidin-4-yl)amino)-triazin-2-yl)-4,7-diazadecane-1,10-diamine.

These light stabilizers may be used singly or in combination of two or more types thereof.

The antioxidant in the first additive and the second additive described later is preferably at least one selected from phosphorus-based antioxidants, sulfur-based antioxidants, and phenol-based antioxidants, and the light stabilizer is preferably at least one selected from ultraviolet absorbers and HALS.

In addition, the first additive and the second additive described below are preferably at least one selected from the group consisting of phosphorus-based antioxidants, sulfur-based antioxidants, phenol-based antioxidants, ultraviolet absorbers, and HALS.

The total content value of antioxidants and light stabilizers (first additives) that can be contained in the catheter tip resin layer relative to the entire catheter tip resin layer may be 0% by mass to 3% by mass, and is preferably 0% by mass or more and less than 3% by mass. When the total content value of antioxidants and light stabilizers is within the above range, floating of antioxidants and light stabilizers on the surface of the resin can be suppressed. In one embodiment, the total content value of antioxidants and light stabilizers that may be contained in the catheter tip resin layer relative to the entire catheter tip resin layer may be 3% by mass or less (lower limit: 0% by mass), less than 3% by mass, 1% by mass or less, 0.5% by mass or less, 0.1% by mass or less, 0.05% by mass or less, 0.02% by mass or less, or 0.01% by mass or less.

In addition, the total content value of at least one selected from the group consisting of phosphorus-based antioxidants, sulfur-based antioxidants, phenol-based antioxidants, ultraviolet absorbers, and HALS, which may be contained in the catheter tip resin layer, relative to the entire catheter tip resin layer, may be 0% by mass to 3% by mass, 0% by mass or more and less than 3% by mass, 0% by mass or more and 1% by mass or less, 0% by mass or more and 0.5% by mass or less, 0% by mass or more and 0.1% by mass or less, 0% by mass or more and 0.05% by mass or less, 0% by mass or more and 0.02% by mass or less, or 0% by mass or more and 0.01% by mass or less.

Other additives besides antioxidants and light stabilizers can also be included in the catheter tip resin layer. These additives can provide advantageous properties to the catheter tip, such as operability.

Examples of other additives in the present specification include, but are not limited to, pigments, plasticizers, lubricants, surfactants, antistatic agents, fillers, diluents, coupling agents, flame retardants, and foaming agents.

Examples of pigments include, but are not limited to, carbon black, lead sulfide, white carbon, titanium white, lithopone, benigara, antimony sulfide, chromium yellow, chromium green, phthalocyanine green, cobalt blue, phthalocyanine blue, and molybdenum orange.

Examples of plasticizers include, but are not limited to: benzoate esters such as diethylene glycol dibenzoate; phthalate esters such as dibutyl phthalate (DBP), di-2-ethylhexyl phthalate (DOP), diisononyl phthalate (DINP), diisodecyl phthalate (DIDP), diundecyl phthalate (DUP), ditridecyl phthalate (DTDP), bis(2-ethylhexyl) terephthalate (DOTP), and bis(2-ethylhexyl) isophthalate (DOIP); aliphatic dicarboxylic acid esters such as di-2-ethylhexyl adipate (DOA), diisononyl adipate (DINA), diisodecyl adipate (DIDA), di-2-ethylhexyl sebacate (DOS), and diisononyl sebacate (DINS); trimellitate

esters such as tri-2-ethylhexyl trimellitate (TOTM), triisononyl trimellitate (TINTM), and triisodecyl trimellitate (TIDTM); pyromellitate esters such as tetra-2-ethylhexyl pyromellitate (TOPM); phosphate esters such as tri-2-ethylhexyl phosphate (TOP) and tricresyl phosphate (TCP); alkyl esters of polyhydric alcohols such as esters of pentaerythritol; epoxidized vegetable oils such as epoxidized soybean oil and epoxidized linseed oil; epoxy esters such as di-2-ethylhexyl ester of 4,5-epoxy-1,2-cyclohexanedicarboxylic acid; alicyclic dicarboxylic acid esters such as diisononyl 1,2-cyclohexanedicarboxylate (DINCH) and di-2-ethylhexyl ester of 4-cyclohexene-1,2-dicarboxylic acid; fatty acid glycol esters such as 1,4-butanediol dicaprate; citrate esters such as tributyl acetylcitrate (ATBC), trihexyl acetylcitrate (ATHC), triethylhexyl acetylcitrate (ATEHC), and trihexyl butyrylcitrate (BTHC); isosorbide diesters; chlorinated paraffins such as chlorinated paraffin wax and chlorinated n-paraffin; chlorinated fatty acid esters such as chlorinated stearic acid esters; and higher fatty acid esters such as butyl oleate.

Examples of lubricants include, but are not limited to: silicones, liquid paraffin, paraffin wax, metal salts of fatty acids such as metal stearates and metal laurates, fatty acid amides, fatty acid waxes, and higher fatty acid waxes.

Examples of surfactants include, but are not limited to: fatty acid esters of glycerin such as glycerin monostearate, glycerin monolaurate, glycerin monooleate, and glycerin monopalmitate; fatty acid esters of propylene glycol such as propylene glycol monolaurate, propylene glycol monostearate, propylene glycol monooleate, and propylene glycol monopalmitate; and fatty acid esters of sorbitan such as sorbitan monolaurate, sorbitan monostearate, sorbitan monooleate, and sorbitan monopalmitate.

Examples of antistatic agents include, but are not limited to: anionic antistatic agents such as alkyl sulfonate type, alkyl ether carboxylic acid type, or dialkyl sulfosuccinate type; nonionic antistatic agents such as polyethylene glycol derivatives, sorbitan derivatives, and diethanolamine derivatives; cationic antistatic agents such as quaternary ammonium salts like alkylamide amine type and alkyl dimethylbenzyl type, and organic acid salts or hydrochlorides of alkylpyridinium type; and amphoteric antistatic agents such as alkyl betaine type and alkyl imidazoline type.

Examples of diluents include, but are not limited thereto: 2,2,4-trimethyl-1,3-pentanediol diisobutyrate and low-boiling aliphatic or aromatic hydrocarbons.

Examples of fillers include, but are not limited to: metal oxides such as calcium carbonate, silica, alumina, clay, talc, diatomaceous earth, and ferrite; fibers and powders of glass, carbon, and metals; glass beads; graphite; aluminum hydroxide; barium sulfate; magnesium oxide; magnesium carbonate; magnesium silicate; and calcium silicate.

Examples of flame retardants include, but are not limited to: inorganic compounds such as aluminum hydroxide, antimony trioxide, magnesium hydroxide, and zinc borate; phosphorus-based compounds such as cresyl diphenyl phosphate, tris(2-chloroethyl) phosphate, tris(1-chloropropyl) phosphate, and tris(1,3-dichloropropyl) phosphate; and halogen-based compounds such as chlorinated paraffin.

Examples of foaming agents include, but are not limited to: organic foaming agents such as azodicarbonamide and oxybisbenzenesulfonyl hydrazide; and inorganic foaming agents such as sodium bicarbonate.

Further, the catheter tip may be provided with an inner layer, in addition to the catheter tip resin layer, such that at least the part, which is in contact with these devices, has low friction when a treatment catheter, guide wire, or other device is inserted into the lumen 10H (the inner layer 11 in Fig. 2). The inner layer is as described above.

(Catheter Main Body Portion)

The catheter main body portion 10 is formed of a hollow tubular member provided with flexibility. The catheter main body portion 10 has a lumen 10H over the entire length of the catheter main body portion 10. The lumen 10H is open at a distal end of the catheter tip 20.

In the aspect shown in Fig. 2, the catheter main body portion 10 includes the inner layer 11 disposed on the inner surface side; the catheter main body portion resin layer(outer layer, referred to as "catheter main body portion resin layer" and "resin layer of catheter main body portion" in the present specification) 12 disposed on the outer periphery of the inner layer 11; the hydrophilic lubricating layer 14 disposed on at least the distal end side of the outer periphery of the catheter main body portion resin layer 12; the metal reinforcing body 13 disposed inside the catheter main body portion resin layer 12; and the contrast marker portion 15 positioned at the most distal end of the catheter main body portion. In the present invention, the content of tungsten carbide in the catheter tip resin layer is less than 50% by mass, and since the X-ray contrast ability is not high compared to the case where tungsten carbide is 50% by mass or more, it is also a preferable aspect to provide other contrast means (for example, the marker portion 15 shown in Fig. 2).

In the aspect shown in Fig. 2, the inner layer 11 and the hydrophilic lubricating layer 14 are disposed extending to the distal end of the catheter tip 20. Therefore, the inner layer 11 and the hydrophilic lubricating layer 14 are common to the inner layer and the hydrophilic lubricating layer in the catheter tip 20.

Examples of the resin constituting the catheter main body portion resin layer 12 include polyolefins such as polypropylene, ethylene-propylene copolymer, and ethylenevinyl acetate copolymer, thermoplastic resins such as modified polyolefin resin, soft polyvinyl chloride, various elastomers such as polyurethane elastomer, polyamide elastomer, and polyester elastomer, and crystalline plastics such as polyamide, crystalline polyethylene, and crystalline

polypropylene. The catheter main body portion resin layer may be a single layer or a plurality of layers of two or more layers. In the case of a plurality of layers, the composition (resin type, mixing ratio, or the like) may be different or the same.

Among the resins, polyamide and/or polyamide elastomer (polyamide, polyamide elastomer, or a combination thereof) is preferred as the resin constituting the catheter main body portion resin layer 12.

The polyamide that may be contained in the catheter main body portion resin layer may be the polyamide described in the section of the above-mentioned catheter tip resin layer.

The polyamide elastomer that may be contained in the catheter main body portion resin layer may be the polyamide elastomer described in the section of the above-mentioned catheter tip resin layer.

The Shore D hardness of the polyamide elastomer used for the catheter main body portion is preferably 30 to 80D and more preferably 40 to 75D from the viewpoint of flexibility. The method for measuring the hardness of polyamide elastomers is as described above. The Shore D hardness of the resin in the catheter main body portion is preferably equal to or greater than the Shore D hardness of the resin in the catheter tip, and is more preferably greater.

In one embodiment, the resin may be included from 20 to 99.9% by mass, more preferably from 30 to 95% by mass, even more preferably from 50 to 90% by mass, and even more preferably from 70 to 90% by mass relative to the entire catheter main body portion resin layer. When the resin content is within the above range, the moldability of the catheter main body portion can be maintained, and particularly, operability during insertion is excellent.

Tungsten carbide may or may not be included in the catheter main body portion resin layer as an X-ray opaque material to identify the position of the catheter main body portion during X-ray irradiation. In one embodiment, the content of tungsten carbide may be 1% by mass or more, 10% by mass or more, 30% by mass or more, or 60% by mass or more of the entire catheter main body portion resin. In one embodiment, the content of tungsten carbide relative to the total catheter main body portion resin layer can be 79.9% by mass or less, 50% by mass or less, 20% by mass or less, 10% by mass or less, 5% by mass or less, or 1% by mass or less (lower limit: 0% by mass).

In one embodiment, the content of tungsten carbide, which may be contained in the catheter main body portion resin layer relative to the entire catheter main body portion resin layer, may be less than the content of tungsten carbide that may be contained in the catheter tip resin layer relative to the entire catheter tip resin layer.

In one embodiment, the catheter main body portion resin layer contains elemental metallic tungsten (W) powder as the X-ray opaque material in order to identify the position of the catheter main body portion during X-ray irradiation. The content of the elemental metallic tungsten powder relative to the total catheter main body portion resin is, for example, more than 0.01% by mass and less than 10% by mass, and may be 0.1% by mass or more and 1% by mass or less. In one embodiment, the catheter main body portion resin layer does not contain elemental metallic tungsten (W).

In addition, the catheter main body portion also includes at least one type of an antioxidant and a light stabilizer to prevent resin deterioration. In one embodiment, at least one type of an antioxidant and a light stabilizer (second additive) may be contained in the catheter main body portion resin layer. The antioxidant and light stabilizer that may be contained in the catheter main body portion resin layer are as described above. The second additive may be the same type as the first additive, or may be a different type.

In one embodiment, the total content value of antioxidants and light stabilizers that may be contained in the catheter main body portion resin layer relative to the entire catheter main body portion resin layer may preferably be 0.1 to 10% by mass, more preferably 0.1 to 3% by mass, still more preferably 0.1 to 1% by mass, and even more preferably 0.1 to 0.5% by mass. In one embodiment, the total content value of antioxidants and light stabilizers that may be contained in the catheter main body portion resin layer relative to the entire catheter main body portion resin layer may be 0.1% by mass or more, 0.15% by mass or more, 0.2% by mass or more, 0.3% by mass or more, 0.5% by mass or more, 1% by mass or more, 2.0% by mass or more, or 3% by mass or more. In one embodiment, the total content value of antioxidants and light stabilizers that may be contained in the catheter main body portion resin layer relative to the entire catheter main body portion resin layer may be 10% by mass or less, 5% by mass or less, 3% by mass or less, 1% by mass or less, 0.5% by mass or less, 0.2% by mass or less, 0.15% by mass or less, 0.11% by mass or less, or 0.1% by mass or less.

In addition, the total content value of at least one type selected from the group consisting of the phosphorus-based antioxidants, the sulfur-based antioxidants, the phenol-based antioxidants, the ultraviolet absorbers, and HALS that may be contained in the catheter main body portion resin layer relative to the entire catheter main body portion resin layer is preferably 0.1 to 10% by mass, more preferably 0.1 to 3% by mass, even more preferably 0.1 to 1% by mass, and still more preferably 0.15 to 0.5% by mass.

The total content value of the antioxidant and the light stabilizer (first additive) in the catheter tip resin layer relative to the catheter tip resin layer may be less than the total content value of the antioxidant and the light stabilizer (second additive) in the catheter main body portion resin layer relative to the entire catheter main body portion resin layer. In one embodiment, the total content value of the antioxidant and the light stabilizer in the catheter tip resin layer relative to the total content value of the antioxidant and the light stabilizer in the entire catheter main body portion resin layer is preferably 1/2 or less, more preferably 1/3 or less, and even more preferably 1/10 or less. As one example, the total content value of the antioxidant and the light stabilizer in the catheter tip resin layer relative to the catheter tip resin layer is 0% by mass, and the total content value of the antioxidant and the light stabilizer in the catheter main body portion resin layer relative to the

entire catheter main body portion resin layer is 0.1% by mass or more. As one example, the total content value of the antioxidant and the light stabilizer in the catheter tip resin layer relative to the catheter tip resin layer is 0% by mass, and the total content value of the antioxidant and the light stabilizer in the catheter main body portion resin layer relative to the entire catheter main body portion resin layer is 0.1% by mass.

In addition, other additives in addition to the antioxidants and the light stabilizers may also be contained in the catheter main body portion resin layer. The other additives that may be contained in the catheter main body portion resin layer are as described above.

Furthermore, the catheter main body portion 10 includes the metal reinforcing body 13. The metal reinforcing body 13 includes a plurality of reinforcing wires that reinforce the catheter main body portion 10. Examples of the reinforcing wires include spiral or braided reinforcing wires. The reinforcing wire is made of metal such as stainless steel. As specific examples, reinforcing wires are formed by crushing stainless steel wires into a flat plate shape to make the thickness of the catheter main body portion 10 thin in the radial direction, and by spiraling or weaving (braided body) a plurality of approximately 8 to 32 stainless steel wires. It is preferable that the number of the reinforcing wires is set to a multiple of 8 to implement reinforcement in a tubular shape in a well-balanced manner.

By making the reinforcing wire into a flat plate, compared to an ellipse, force is received uniformly against external stress, resulting in consistent physical properties.

The catheter main body portion 10 may also include the inner layer 11 and the hydrophilic lubricating layer 14. The inner layer 11 and the hydrophilic lubricating layer 14 are as described above.

It should be noted that the number of the layers constituting the catheter main body portion 10 or the constituent material of each layer may be different along the longitudinal direction of the catheter main body portion 10. For example, in order to provide greater flexibility, the part on the distal end side of the catheter main body portion 10 can have a reduced number of layers or use more flexible materials.

In the present specification, the catheter main body portion refers to the part having the metal reinforcing body 13 in any radial direction, and the catheter tip refers to all parts on the distal end side relative to the catheter main body portion. The distal end portion of the catheter main body portion 10 and the catheter tip 20 can be joined, for example, by thermal fusion, thereby being integrated.

## Examples

The effects of the present invention will be described with reference to the following Examples and Comparative Examples. Note that the technical scope of the present invention is not limited only to the following Examples. Note that, in the following Examples, unless otherwise specified, each operation was performed at room temperature (25°C). In addition, unless otherwise specified, "%" or "parts" means "% by mass" or "parts by mass".

### [Preparation of Dumbbell-Shaped Test Pieces]

### [Example 1]

60 parts by mass of polyamide elastomer (Shore D hardness: 40, weight average molecular weight (Mw): approximately 40,000) was mixed with 40 parts by mass (mass ratio) of tungsten carbide particles (average particle diameter: 3.0 to 4.19 μm). The obtained resin was subjected to press molding using a 20-ton manual hydraulic heating press at a set temperature of 200°C. The thickness of the molded product was set to 1.9 to 2.1 mm. Dumbbell-shaped test pieces were prepared by punching the molded press sheet according to JIS K 7161-2:2014 5A.

### [Comparative Example 1]

Dumbbell-shaped test pieces were prepared in the same manner as in Example 1, except that tungsten particles (average particle diameter: 3.0 um, W content: 99.9% by mass or more) were used instead of tungsten carbide particles.

### [Reference Example 1]

Dumbbell-shaped test pieces were prepared in the same manner as in Example 1, except that 30 parts by mass of polyamide elastomer (Shore D hardness: 40, weight average molecular weight (Mw): approximately 40,000) were mixed with 70 parts by mass (mass ratio) of tungsten carbide particles (average particle diameter: 3.0 to 4.19 μm).

### [Load Test]

The dumbbell-shaped test pieces prepared in the examples and comparative examples were left to stand under

high temperature and high humidity conditions (80°C, relative humidity: 95% RH) for 21 hours.

[Tensile Test]

A tensile test was conducted on the dumbbell-shaped test pieces of Example 1 and Comparative Example 1 before and after the load test in accordance with JIS K 7161-1:2014.

<Examination Conditions>

Distance between grips: 50 mm
Distance between marked lines: 20 mm
Test speed: 100 mm/min

As an indicator of durability, the residual ratio shown in the following formula was calculated. The higher the residual ratio, the greater the durability. Here, the fracture stroke length refers to the value obtained by subtracting the gauge length before the test from the distance between the marked lines after fracture.

[Math. 2]

$$\text{Residual ratio (\%)} = B/A \times 100$$

A: Fracture stroke length before load test (mm)
B: Fracture stroke length after load test (mm)

[Table 2]

Table 2: Tensile test results of Example 1 and Comparative Example 1 before and after load test

| Condition name | Fracture stroke length [mm] | | Residual ratio [%] = B/A |
|---|---|---|---|
| | No load (A) | After 80°C x 95% RH 21 hours (B) | |
| Tungsten mixed sample Tungsten carbide mixed sample (40% by weight) | 368.1<br>281.6 | 65.9<br>257.3 | 17.9<br>91.4 |

As a result of the tensile test, the dumbbell-shaped test piece of Comparative Example 1 showed a residual ratio of 17.9% before and after the load test, whereas the dumbbell-shaped test piece of Example 1 showed a higher residual ratio of 91.4% before and after the load test, indicating a higher residual ratio than that of Comparative Example 1. From this result, it was confirmed that the dumbbell-shaped test piece of Example 1 has higher durability than that of Comparative Example 1, even without containing a first additive selected from the group consisting of an antioxidant and a light stabilizer. Further, the fracture stroke length of Reference Example 1 under no load was 178.6 mm, indicating that the flexibility is high due to the tungsten carbide content being less than 50% by mass.

The present application is based on Japanese Patent Application No. 2023-102520 filed on June 22, 2023, the entire content of which is incorporated herein by reference.

Reference Signs List

1 Catheter
10 Catheter main body portion
30 Hub
20 Catheter tip
40 Kink-resistant protector (strain relief)

## Claims

1. A catheter comprising:

   a catheter tip having a catheter tip resin layer containing 1% by mass or more and less than 50% by mass of tungsten carbide (WC); and
   a catheter main body portion having a catheter main body portion resin layer containing at least one type of second additive selected from the group consisting of antioxidants and light stabilizers,
   wherein the total content value of at least one type of first additive selected from the group consisting of antioxidants and light stabilizers contained in the catheter tip resin layer is less than the total content value of the second additive in the catheter main body portion resin layer, and
   the total content value of the first additive relative to the catheter tip resin layer is 0% by mass or more and less than 3% by mass.

2. The catheter according to claim 1, wherein the resin contained in the catheter tip resin layer is a resin having ester bonds and/or amide bonds.

3. The catheter according to claim 2, wherein the resin having ester bonds and/or amide bonds contains a polyamide-based resin.

4. The catheter according to claim 1 or 2, wherein the particle diameter of the tungsten carbide is 1 to 10 $\mu$m.

5. The catheter according to claim 1 or 2, wherein the catheter tip does not contain elemental tungsten (W).

6. The catheter according to claim 1 or 2, wherein

   the antioxidant is at least one selected from phosphorus-based antioxidants, sulfur-based antioxidants, and phenol-based antioxidants, and
   the light stabilizer is at least one selected from ultraviolet absorbers and HALS.

7. The catheter according to claim 1 or 2, wherein the catheter main body portion includes a contrast marker portion.

FIG. 1

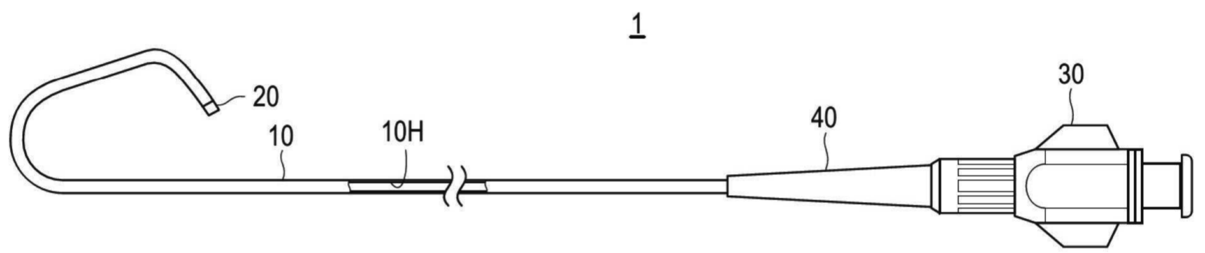


FIG. 2

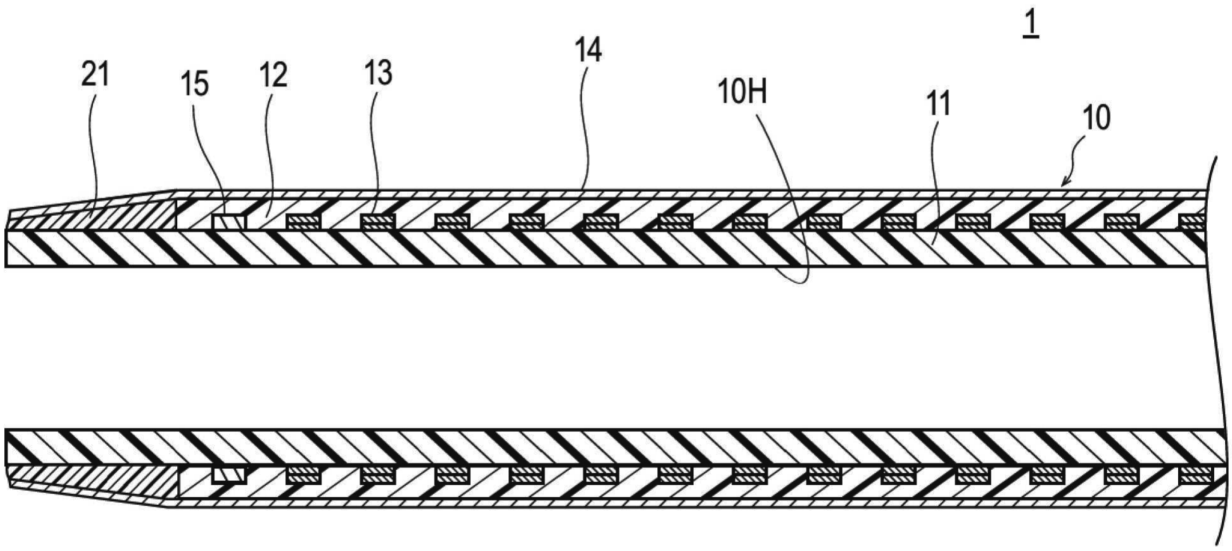

# INTERNATIONAL SEARCH REPORT

International application No.
**PCT/JP2024/021171**

## A. CLASSIFICATION OF SUBJECT MATTER

***A61M 25/098***(2006.01)i; ***A61L 29/02***(2006.01)i; ***A61L 29/06***(2006.01)i; ***A61L 29/14***(2006.01)i; ***C08K 3/014***(2018.01)i; ***C08L 77/00***(2006.01)i
FI: A61M25/098; C08K3/014; C08L77/00; A61L29/02; A61L29/06; A61L29/14

According to International Patent Classification (IPC) or to both national classification and IPC

## B. FIELDS SEARCHED

Minimum documentation searched (classification system followed by classification symbols)

A61M25/098; A61L29/02; A61L29/06; A61L29/14; C08K3/014; C08L77/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

## C. DOCUMENTS CONSIDERED TO BE RELEVANT

| Category* | Citation of document, with indication, where appropriate, of the relevant passages | Relevant to claim No. |
|---|---|---|
| X | US 2017/0313853 A1 (MEDTRONIC, INC.) 02 November 2017 (2017-11-02) claims 1-30, paragraphs [0001]-[0313], fig. 1-9 | 1-6 |
| Y | claims 1-30, paragraphs [0001]-[0313], fig. 1-9 | 7 |
| X | JP 2007-508056 A (MEDTRONIC, INC.) 05 April 2007 (2007-04-05) claims 1-99, paragraphs [0001]-[0015], [0041]-[0042], fig. 1-3 | 1-6 |
| Y | claims 1-99, paragraphs [0001]-[0015], [0041]-[0042], fig. 1-3 | 7 |
| Y | JP 2009-268648 A (NIPRO CORPORATION) 19 November 2009 (2009-11-19) paragraphs [0016]-[0036], fig. 1-2 | 7 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 August 2024** | **13 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/021171**

| Patent document cited in search report | Publication date (day/month/year) | Patent family member(s) | Publication date (day/month/year) |
|---|---|---|---|
| US 2017/0313853 A1 | 02 November 2017 | WO 2017/189761 A1<br>EP 3448449 A1<br>CN 109069700 A | |
| JP 2007-508056 A | 05 April 2007 | US 2004/0073158 A1<br>claims 1-99, paragraphs [0001]-[0030], [0051]-[0052], fig. 1-3<br>WO 2005/035043 A2 | |
| JP 2009-268648 A | 19 November 2009 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5584821 A
- US 4100309 A
- JP 59019582 A
- JP 2023102520 A